# EUROPEAN PATENT APPLICATION

(11) **EP 2 382 969 A1**
(43) Date of publication of application: **02.11.2011**
(21) Application number: 11164326.8
(22) Date of filing: 29.04.2011
(51) Int. Cl.: A61K 9/16, A61K 9/50, A61K 31/165

(54) **Multicoated Aliskiren formulations**

(30) Priority: 30.04.2010 TR 201003449
(71) Applicant: Sanovel Ilac Sanayi ve Ticaret A.S., 34398 Istanbul (TR)
(72) Inventor: Cifter, Ümit, 34398, Istanbul (TR); Yelken, Gülay, 34398, Istanbul (TR); Türkyilmaz, Ali, 34398, Istanbul (TR)
(74) Representative: Sevinç, Erkan

(57) **Abstract**

An oral pharmaceutical formulation of aliskiren or a pharmaceutically acceptable salt or polymorph thereof, comprising at least two coating layers.

## Description

### Field of Invention

The present invention relates to formulations of aliskiren or a pharmaceutically acceptable salt of aliskiren. The present invention more particularly relates to stable multicoated formulations of aliskiren with desired levels of solubility and dissolution rate.

### Background of Invention

Aliskiren, with the chemical name (2S,4S,5S,7S)-5-amino-N-(2-carbamoyl-2-methylpropyl)-4-hydroxy-2-isopropyl-7-[4-methoxy-3-(3-methoxypropoxy)benzyl]-8-methylnonamide, is the first orally-active renin inhibitor with a non-peptide structure. Its chemical structure is illustrated with Formula I given below.

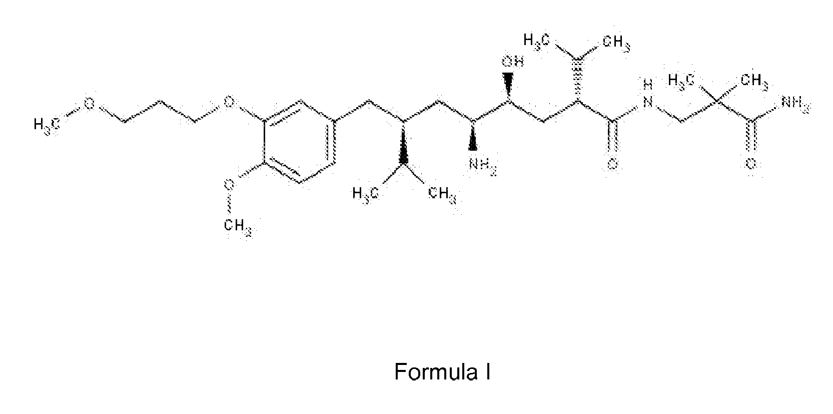

The patent US5559111 discloses the aliskiren molecule together with δ-amino-γ-hydroxy-ω-aryl-alkanoic acid amide derivatives.

The patent EP1200390 discloses intermediates preparing the aliskiren molecule and processes for their preparation.

The patent application WO2009143423A1 discloses aliskiren monofumarate and a process for the preparation thereof.

The patent application W02009149344 discloses a solid state of aliskiren free base.

The patent application W02009040373 discloses a roller compacted solid oral dosage form comprising aliskiren in an amount of more than 38% by weight based on the total weight of the dosage form.

The patent application W02005089729 discloses a solid oral dosage form comprising aliskiren in an amount of more than %46 by weight based on the total weight thereof.

Aliskiren hemifumarate is a white to bright yellowish crystalline powder. It is freely soluble in phosphate buffer, n-octanol, and water. Due to its physicochemical structure, however, obtaining a stable oral formulation of aliskiren is quite difficult. Water contact of aliskiren results in changes in the polymorphic structure, frequently leading to stability problems. Aliskiren is also quite hygroscopic. Its contact with water must not be avoided only efficiently during its production phase, but also when it is in its finished formulation form.

Whilst single-layer coatings are applied on already-finished formulations, this does not suffice in providing protection against humidity, and therefore blisters, containing aluminum to avoid humidity, are used as well. Blisters, however, are of high costs and thus not economic.

Carrying out the film coating operation at desired accuracy levels is very crucial with respect to ensuring the stability of aliskiren molecule. A preferred coating must both provide protection against environmental effects to ensure stability, and not lead to problems encountered in film-coating processes, such as wrinkling surfaces, blister and bubble formation, efflorescence, peeling, etc.

Considering such problems, it is obvious that a novelty is required in the relevant art of formulations comprising aliskiren.

### Object and Brief Description of Invention

The present invention provides an aliskiren formulation, eliminating all aforesaid problems and bringing additional advantages to the relevant prior art.

Accordingly, the main object of the present invention is to obtain a formulation of aliskiren, which is stable and has desired levels of solubility and dissolution rate.

Another object of the present invention is to develop a multi-coating, providing the stability of aliskiren-containing formulation and not affecting the solubility and dissolution rates thereof.

A further object of the present invention is to develop a process, enabling aliskiren production without influencing the solubility and dissolution rate thereof.

Still a further object of the present invention is to provide a coating operation, efficiently avoiding environmental effects and allowing coating the formulation in a faultless manner.

A pharmaceutical formulation comprising aliskiren or a pharmaceutically acceptable salt or polymorph of aliskiren has been developed to carry out all objects, referred to above and to emerge from the following detailed description.

According to a preferred embodiment of the present invention, said novelty is developed with at least two outer coating layers. One of said coating layers contains hydroxypropyl methyl cellulose, whereas the other contains polyvinyl alcohol. Said hydroxypropyl methyl cellulose is low-substituted hydroxypropyl methyl cellulose.

According to a preferred embodiment of the present invention, the amount of aliskiren in said oral tablet formulation is not more than 44% by weight and is preferably 36% by weight.

According to another preferred embodiment of the present invention, at least one or a properly-proportioned mixture of pullulan or trehalose is used as a binder.

According to another preferred embodiment of the present invention, at least one or a properly-proportioned mixture of croscarmellose sodium, crospovidone, and sodium starch glycolate is used as a disintegrant.

According to a preferred embodiment of the present invention, the weight ratio of pullulan to croscarmellose sodium is between 0.02 to 25, preferably 0.1 to 10, and more preferably 0.1 to 3.

According to a preferred embodiment of the present invention, at least one or a properly-proportioned mixture of colloidal silicone dioxide, talc, and aluminum silicate is used as a glidant.

According to a preferred embodiment of the present invention, said glidant is preferably colloidal silicone dioxide.

According to a preferred embodiment of the present invention, magnesium stearate is used as a lubricant.

A further preferred embodiment according to the present invention provides a method for preparing a pharmaceutical formulation, this method comprising the steps of
a. preparing an alcoholic or hydroalcoholic granulation solution of pullulan,
b. adding aliskiren, half of croscarmellose sodium, and corn starch into this solution and mixing the latter,
c. blending said granulation solution and powder mixture in a high-shear granulator in order to form granules,
d. sieving such obtained wet granules, thereafter drying and then sieving the same,
e. sieving and then adding half of croscarmellose sodium and colloidal silicone dioxide into this mixture and mixing the latter until a homogeneous mixture is formed,
f. sieving and then adding magnesium stearate into the powder mixture obtained and mixing it for a short time,
g. compacting the final powder mixture to provide tablets,
h. preparing a coating suspension of hydroxypropyl methyl cellulose-containing coating material in alcohol, and then subjecting the core tablets to preliminary coating process,
i. preparing a coating suspension of polyvinyl alcohol-containing coating material and carrying out the final coating process with this coating suspension.

In a further preferred embodiment of the present invention, said pharmaceutical formulation consisting of
a. core
   a. aliskiren or a pharmaceutically acceptable salt or polymorph thereof at 5 to 44% by weight,
   b. starch at 5 to 85% by weight,
   c. pullulan at 0.25 to 10% by weight,
   d. croscarmellose sodium at 0.2 to 10% by weight,
   e. colloidal silicone dioxide at 0.1 to 5% by weight,
   f. magnesium stearate at 0.2 to 10% by weight;
b. coating
   a. hydroxymethyl propyl cellulose at 0.5 to 5% by weight (lower coating layer),
   b. polyvinyl alcohol at 0.5 to 5% by weight (upper coating layer).

### Detailed Description of Invention

### Example 1

| **Unit Formula** | **Amount in tablet (%)** |
|---|---|
| **Core tablet** | **100** ¹⁰ |
| Aliskiren hemifumarate | 42 |
| Cornstarch | 49.5 |
| Pullulan | 3 |
| Croscarmellose sodium | 4 |
| Silicone dioxide | 0.5 |
| Magnesium stearate | 1 |
| **Film coating** | **5** |
| HPMC (lower coating layer) | 2 |
| Opadry-AMB (upper coating layer) | 3 |

First of all, an alcoholic or hydroalcoholic granulation solution of pullulan is prepared. Then aliskiren, half of croscarmellose sodium, and corn starch are added into this solution and the latter is mixed. The granulation solution prepared above and powder mixture are blended in a high-shear granulator in order to form granules. Such obtained wet granules are sieved, thereafter dried and then sieved again. Then the remaining half of croscarmellose sodium and colloidal silicone dioxide are sieved and then added into this mixture and the latter is mixed until a homogeneous mixture is formed. Magnesium stearate is sieved and then added into the powder mixture obtained and it is mixed for a short time. The powder mixture obtained is compacted to form tablets. Then, a coating suspension of hydroxypropyl methyl cellulose-containing coating material in alcohol is prepared and the core tablets are accordingly subjected to a preliminary coating process. At the final step, a coating suspension of polyvinyl alcohol-containing coating material is prepared and the final coating process is performed accordingly. The coating material containing polyvinyl alcohol is opadry-amb and comprises talk and titanium dioxide, besides polyvinyl alcohol.

### Example 2

| **Unit Formula** | **Amount in tablet (%)** |
|---|---|
| **Core tablet** | **100** |
| Aliskiren hemifumarate | 42 |
| Cornstarch | 29.5 |
| Mannitol | 20 |
| Pullulan | 3 |
| Croscarmellose sodium | 4 |
| Silicone dioxide | 0.5 |
| Magnesium stearate | 1 |
| **Film coating** | **5** |
| HPMC (lower coating layer) | 2 |
| Opadry-AMB (upper coating layer) | 3 |

With the invention realized, stable aliskiren formulations can be obtained which have surprisingly good solubility and dissolution rates, and thus high bioavailability. The formulation developed is made with at least two outer coating layers. The outermost upper coating layer contains polyvinyl alcohol. The lower coating layer contains low-substituted hydroxypropyl methyl cellulose. Pullulan or trehalose, used as binders, protect aliskiren against moist; thereby considerably facilitate the production of a stable formulation. The weight ratio of pullulan to croscarmellose sodium is between 0.02 to 25, preferably 0.1 to 10, and more preferably 0.1 to 3. Desired distribution profiles are obtained in this proportion range.

The formulation obtained is used in treating hypertension.

According to a preferred embodiment of the present invention tablet is coated with at least two coating layers.

It is further possible to use the following additional excipients in the formulation.

Suitable binders include, but are not restricted to, at least one or a mixture of polyvinylprolidone, gelatin, sugars, glucose, natural glue, gums, synthetic celluloses, polymethacrylate, hydroxypropyl methyl cellulose, hydroxypropyl cellulose, carboxymethyl cellulose, methyl cellulose, and other cellulose derivatives.

Suitable glidants include, but are not restricted to, at least one or a mixture of colloidal silicone dioxide, talc, aluminum silicate.

Suitable lubricants include, but are not restricted to, at least one or a mixture of sodium stearil fumarat, magnesium stearate, polyethylene glycol, stearic acid, metal stearates, boric acid, sodium chloride benzoate and acetate, sodium or magnesium lauryl sulfate.

Suitable surface active agents include, but are not restricted to, at least one or a mixture of sodium lauryl sulfate, dioctyl sulfosuccinate, polysorbates and polyoxyethylene alkyl esters and ethers thereof, glyceryl monolaurate saponins, sorbitan laurate, sodium lauryl sulfate, magnesium lauryl sulfate.

Suitable coating agents include, but are not restricted to, hydroxypropyl methyl cellulose, polyethylene glycol, polyvinylprolidone, polyvinylprolidone-vinyl acetate copolymer(PVP-VA), pullulan like polymers, and all kinds of Opadry, as well as pigments, dyes, titanium dioxide and iron oxide, talk.

Suitable colorants include, but are not restricted to, a mixture of food, drug, and cosmetic (FD&C) dyes (FD&C blue, FD&C green, FD&C red, FD&C yellow, FD&C lake), ponceau, indigo drug & cosmetic (D&C) blue, indigotine FD&C blue, carmoisine indigotine (indigo Carmine); iron oxides (e.g. iron oxide red, yellow, black), quinoline yellow, flame red, brilliant red (carmine), carmoisine, sunset yellow.

Suitable preservatives include, but are not restricted to a mixture of methyl paraben and propyl paraben and salts thereof (e.g. sodium or potassium salts), sodium benzoate, citric acid, benzoic acid, butylated hydroxytoluene and butylated hydroxyanisole.

The protection scope of the present invention is set forth in the annexed Claims and cannot be restricted to the illustrative disclosures given above, under the detailed description. Any alternative embodiments to be produced by persons skilled in the art according to the basic principles, which are under the protection scope as set forth in the Claims, shall be an infringement of the present invention.

## Claims

1. A pharmaceutical tablet formulation of aliskiren or a pharmaceutically acceptable salt or polymorph thereof, comprising at least two coating layers.

2. The pharmaceutical tablet formulation according to Claim 1 **characterized in that** tablet is coated with at least two coating layers.

3. The pharmaceutical tablet formulation according to Claim 1, wherein one of said coating layers contains hydroxypropyl methyl cellulose, whereas the other contains polyvinyl alcohol.

4. The pharmaceutical tablet formulation according to any of the preceding claims, wherein the maximum amount of aliskiren in said pharmaceutical tablet formulation is 44% by weight.

5. The pharmaceutical tablet formulation according to any of the preceding claims, further comprising at least one or a mixture of binders, disintegrants, glidants, and lubricants.

6. The pharmaceutical tablet formulation according to any of the preceding claims, comprising at least one or a properly-proportioned mixture of pullulan or trehalose as the binder.

7. The pharmaceutical tablet formulation according any of the preceding claims, comprising at least one or a properly-proportioned mixture of croscarmellose sodium, crospovidone, sodium starch glycolate as the disintegrant.

8. The pharmaceutical tablet formulation according to any of the preceding claims, wherein said disintegrant is preferably croscarmellose sodium.

9. The pharmaceutical tablet formulation according to any of the preceding claims, wherein the weight ratio of pullulan to croscarmellose sodium is between 0.02 to 25, preferably 0.1 to 10, and more preferably 0.1 to 3.

10. The pharmaceutical tablet combination according to any of the preceding claims, further comprising at least one or a properly-proportioned mixture of colloidal silicone dioxide, talk and aluminum silicate as the glidant.

11. The pharmaceutical tablet formulation according to any of the preceding claims, wherein said glidant is preferably colloidal silicone dioxide.

12. The pharmaceutical tablet formulation according to any of the preceding claims, comprising magnesium stearate as the lubricant.

13. A method for preparing a pharmaceutical tablet formulation according to any of the preceding claims, comprising the steps of
a. preparing an alcoholic or hydroalcoholic granulation solution of pullulan,
b. adding aliskiren, half of croscarmellose sodium, and corn starch into this solution and mixing the latter,
c. blending said granulation solution and powder mixture in a high-shear granulator in order to form granules,
d. sieving such obtained wet granules, thereafter drying and then sieving the same,
e. sieving and then adding half of croscarmellose sodium and colloidal silicone dioxide into this mixture and mixing the latter until a homogeneous mixture is formed,
f. sieving and then adding magnesium stearate into the powder mixture obtained and mixing it for a short time,
g. compacting the final powder mixture to provide tablets,
h. preparing a coating suspension of hydroxypropyl methyl cellulose-containing coating material in alcohol, and then subjecting the core tablets to preliminary coating process,
i. preparing a coating suspension of polyvinyl alcohol-containing coating material and carrying out the final coating process with this coating suspension.

14. The pharmaceutical tablet formulation according to any of the preceding claims, consisting of
a. core
a. aliskiren or a pharmaceutically acceptable salt or polymorph thereof at 5 to 44% by weight,
b. starch at 5 to 85% by weight,
c. pullulan at 0.25 to 10% by weight,
d. croscarmellose sodium at 0.2 to 10% by weight,
e. colloidal silicone dioxide at 0.1 to 5% by weight,
f. magnesium stearate at 0.2 to 10% by weight;
b. coating
a. hydroxypropyl methyl cellulose at 0.5 to 5% by weight,
b. polyvinyl alcohol at 0.5 to 5% by weight.

15. The pharmaceutical tablet formulation according to any of the preceding claims for preventing or treating hypertension in mammalians and particularly in humans.
